# EUROPÄISCHE PATENTSCHRIFT

(11) **EP 0 614 869 B1**
(45) Veröffentlichungstag und Bekanntmachung des Hinweises auf die Patenterteilung: **21.05.1997**
(21) Anmeldenummer: 94102941.5
(22) Anmeldetag: 26.02.1994
(51) Int. Cl.: C07C 45/40, B01J 35/04

(54) **Hydrogenolytische Reduktion peroxidischer Ozonolyseprodukte**
Hydrogenolytic reduction of peroxidic ozonolysis compounds
Réduction hydrogénolytique des composés peroxydiques d'ozonolyse

(30) Priorität: 12.03.1993 AT 484/93
(43) Veröffentlichungstag der Anmeldung: 14.09.1994
(73) Patentinhaber: DSM Chemie Linz GmbH, 4021 Linz (AT)
(72) Erfinder: Pollhammer, Stefan, A-4030 Linz (AT); Schaller, Josef, A-4020 Linz (AT); Winetzhammer, Willibald, Dipl.-Ing. Dr., A-4641 Steinhaus (AT)
(74) Vertreter: Kunz, Ekkehard, Dr.

(56) Entgegenhaltungen:
- EP-A- 0 146 784
- EP-A- 0 147 593
- AT-A- 379 799

## Beschreibung

Die Ozonolyse von Olefinen liefert auf umweltfreundliche Weise Carbonylverbindungen, wie Aldehyde oder Ketone, oder, je nach den Aufarbeitungsbedingungen, deren Halbacetale, Acetale oder Ketale, die wertvolle Ausgangsstoffe in der präparativen, organischen Chemie darstellen. Bei der Ozonolyse entstehen bekanntlich aber auch Peroxide, die erst durch Reduktion in die gewünschten Produkte übergeführt werden können. Verfahren zur Herstellung von Carbonylverbindungen, deren Halbacetalen, Acetalen oder Ketalen durch Ozonolyse und Reduktion, die in technischem Maßstab durchgeführt werden, sind in EP-B-0 146 784 oder EP-B-0 147 593 beschrieben. Gemäß der Offenbarung dieser beiden Patentschriften werden Verbindungen, die olefinische Doppelbindungen aufweisen, in einem niedrigen aliphatischen Alkohol bei Temperaturen von -80 °C bis 20 °C mit der äquivalenten Menge Ozon umgesetzt, worauf die peroxidische Reaktionslösung in eine Suspension eines Hydrierkatalysators unter Zugabe von Wasserstoff in einer solchen Weise eingespeist wird, daß in der Reaktionsmischung eine Peroxidkonzentration von 0,1 Mol/l nicht überschritten wird. Da bei dieser Art der Reaktionsführung saure Nebenprodukte entstehen, die den Katalysator vergiften und rasch desaktivieren würden, muß der pH-Wert der Reaktionsmischung durch Zugabe einer Base kontrolliert werden.

Es wurde nun unerwarteterweise gefunden, daß in den obgenannten Verfahren bei Verwendung eines Monolithkatalysators anstelle der herkömmlichen Katalysatorsuspension so gut wie keine sauren Nebenprodukte entstehen, auch wenn derselbe Katalysatorgrundstoff, z.B. Palladium oder Platin verwendet wird. Überdies hat sich bei Vergleichsversuchen herausgestellt, daß bei Verwendung eines Monolithkatalysators höhere Ausbeuten und reinere Produkte entstehen, wobei der Katalysator über lange Zeiträume hochaktiv bleibt.

Gegenstand der Erfindung ist daher ein Verfahren zur hydrogenolytischen Reduktion peroxidischer Ozonolyseprodukte zu den entsprechenden Carbonylverbindungen in einem unter den Reaktionsbedingungen inerten organischen Verdünnungsmittel in Gegenwart eines Katalysators bei einem Wasserstoffdruck von 0,01 bis 2,0 MPa und bei Temperaturen von -10 bis 150 °C, das dadurch gekennzeichnet ist, daß als Katalysator ein Monolithkatalysator eingesetzt wird.

Unter Carbonylverbindungen sind dabei aliphatische, aromatische oder heterocyclische Aldehyde oder Ketone zu verstehen, wobei je nach der Art des, als Ausgangsverbindung zur Ozonolyse verwendeten, Moleküls und je nach der Anzahl von dessen Doppelbindungen meherere Aldehyd- und/oder Ketongruppen in der Carbonylverbindung vorliegen können. Carbonylverbindungen, die mit Hilfe des erfindungsgemäßen Verfahrens herstellbar sind und die entsprechenden, als Ausgangsmaterialien verwendeten Verbindungen sowie die Herstellung entsprechender, peroxidischer Ozonolyseprodukte sind beispielsweise aus EP-B-0 146 784 oder EP-B-0 147 593 bekannt. Für das erfindungsgemaße Verfahren ist die Art und Weise der Herstellung der peroxidischen Ozonolyseprodukte nicht ausschlaggebend. Maßgeblich ist, daß die peroxidischen Ozonolyseprodukte in einem unter den Reaktionsbedingungen der Hydrogenolyse inerten, organischen Verdünnungsmittel zumindest teilweise gelöst vorliegen. Unter organisches Verdünnungsmittel sind dabei übliche, bei der Hydrogenolyse verwendete Verdünnungsmittel zu verstehen, beispielsweise aliphatische oder aromatische, gegebenenfalls chlorierte Kohlenwasserstoffe, wie Pentan, Hexan, Cyclohexan, Toluol, Xylole, Methylenchlorid, Dichlorethan, Chlorbenzole, Carbonsäureester wie Essigsäuremethyl-, -ethyl oder -butylester, Ether wie Diethylether, Diisopropylether, Tetrahydrofuran, Ketone wie Aceton, Methylbutylketon, Alkohole wie Methanol, Ethanol, iso-Propanol. Bei der Verwendung von Alkoholen als Verdünnungsmittel können als Produkte nicht nur die, den eingesetzten Olefinen entsprechenden, Aldehyde oder Ketone, sondern auch deren Halbacetale, Acetale oder deren Ketale entstehen, wobei die Acetalisierung oder Ketalisierung im wesentlichen von den pH-Wert Bedingungen abhängig ist.
Bevorzugt werden im erfindungsgemäßen Verfahren peroxidische Ozonolyselösungen in einem niederen, aliphatischen Alkohol mit 1 bis 6 C-Atomen, ganz besonders bevorzugt Lösungen peroxidischer Ozonolyseprodukte in einem Alkohol, die gemäß einer der in EP-B-0 147 593 beschriebenen Verfahrensweise hergestellt wurden, eingesetzt. Die Konzentration der Peroxide in der Lösung ist für das erfindungsgemäße Verfahren aber überraschenderweise nicht von Bedeutung. Im allgemeinen werden Lösungen peroxidischer Ozonolyseprodukte jedoch so hergestellt, daß die Peroxidkonzentration 1,5 Mol/l nicht übersteigt, da Peroxide in höheren Konzentrationen zur explosionsartigen Zersetzung neigen. Bevorzugt sind daher Lösungen mit einer Peroxidkonzentration, die 1,5 Mol/l nicht übersteigt.

Unter einem Monolithkatalysator ist ein Katalysator zu verstehen, die aus einem Träger, der mit einem Katalysatorgrundstoff beschichtet ist, bestehen. Der Träger besitzt bevorzugt eine möglichst große Oberfläche, die beispielsweise durch waben-oder lamellenförmige Strukturierung erreicht werden kann. Der Träger liegt in einem Stück vor und kann aus dazu geeigneten Materialien, beispielsweise aus Metall, Glas, Keramik, Kunststoff bestehen. Bevorzugt ist ein Metallträger, beispielsweise aus Stahl, Aluminium, da sich gezeigt hat, daß dieser die Reaktionswärme gleichmäßig aufnehmen und wieder in das umgehende Reaktionsmedium abgeben kann. Es hat sich nämlich herausgestellt, daß es bei Verwendung nichtleitender Materialien als Träger zu lokalen Überhitzungen im Reaktionsmedium kommen kann, sodaß Ausbeuten und Reinheit der Reaktionsprodukte negativ beeinflußt werden können.

Als Katalysatorgrundstoff sind bei der Reduktion organischer Peroxidlösungen übliche Katalysatorgrundstoffe zu verstehen. Übliche Katalysatorgrundstoffe sind beispielsweise Edelmetalle wie Platin, Palladium, Übergangsmetalle, wie Nickel, Kobalt, Rhodium, deren Oxide, oder Mischungen solcher Metalle oder Metalloxide. Dabei können diese Metalle durch Schwermetalle wie Blei, Wismuth partiell vergiftet sein. Bevorzugt werden im erfindungsgemäßen Verfahren Edelmetalle oder Mischungen von Edelmetallen mit Übergangsmetallen als Katalysatorgrundstoff eingesetzt. Die Ausbeuten sind beim erfindungsmäßen Verfahren an sich von der eingesetzten Menge des Katalysatorgrundstoffes unabhängig, jedoch empfiehlt es sich zur Erzielung einen ausreichenden Hydrierungsgeschwindigkeit 0,1 bis 5,0 Gew.% bevorzugt 0,2 bis 2,0 Gew.% Katalysatorgrundstoff bezogen auf die Gesamtmenge an peroxidischer Lösung, vorzulegen.

Die Herstellung solcher Katalysatoren kann durch ein übliches Beschichtungsverfahren, beispielsweise durch Aufdampfen des Katalysatorgrundstoffes auf einen Träger oder Imprägnierung des Trägers mit dem Katalysatorgrundstoff erfolgen.

Zur Durchführung des erfindungsgemäßen Verfahrens werden die peroxidischen Ozonolyseprodukte im verwendeten Verdünnungsmittel mit dem Monolithkatalysator und mit Wasserstoff in Kontakt gebracht. Dabei kann der Monolithkatalysator in das, die peroxidischen Ozonolyseprodukte enthaltende, Verdünnungsmittel eingebracht und Wasserstoff unter Rühren zugesetzt werden, oder das, das Ozonolyseprodukt enthaltende Verdünnungsmittel wird zusammen mit Waserstoff kontinuierliche über den Monolithkatalysator geleitet. Bevorzugt erfolgt das Inkontaktbringen kontinuierlich.

Die Reduktion, die im allgemeinen exotherm verläuft, wird bei Temperaturen von -10 bis 150 °C, bevorzugt von 15 bis 70 °C, besonders bevorzugt von etwa Raumtemperatur bis 50 °C ausgeführt. Dabei wird der Wasserstoff durch Einleiten oder Aufdrücken wie üblich zugesetzt. Der Wasserstoffdruck beträgt im allgemeinen 0,01 bis 2,0 MPa, bevorzugt 0,1 bis 1,0 MPa. Der jeweils geeignete Wasserstoffdruck und die jeweils geeignete Temperatur sind dabei für das jeweilige Ozonolyseprodukt leicht durch einen Vorversuch zu ermitteln.

Bei der Reduktion entstehen die, den eingesetzten peroxidischen Ozonolyseprodukten entsprechenden Aldehyde, Ketone bzw. deren Halbacetale, deren Acetale oder deren Ketale. Zur Aufarbeitung wird die hydrierten Produktlösung vom Katalysator, etwa durch Herausnehmen des Katalysatorträgers aus dem Reaktionsgemisch oder durch Abpumpen der Produktlösung aus dem Reaktionsgefäß, das den Katalysator enthält, auf einfache Weise abgetrennt. Eine komplizierte Abtrennung der Katalysatorsuspension durch Filtrieren oder Abzentrifugieren, wie bisher erforderlich, die noch dazu durch das Inkontaktbringen der Katsalysatorsuspension mit atmosphärischem Sauerstoff mit Brandgefahr verbunden ist, entfällt. Das gebildete Hydrogenolyseprodukt wird aus der Reaktionsmischung durch Entfernen des organischen Verdünnungsmittels isoliert und gegebenenfalls nach üblichen Verfahren wie Kristallisations-, Chromatographie- oder Destillationsverfahren gereinigt.

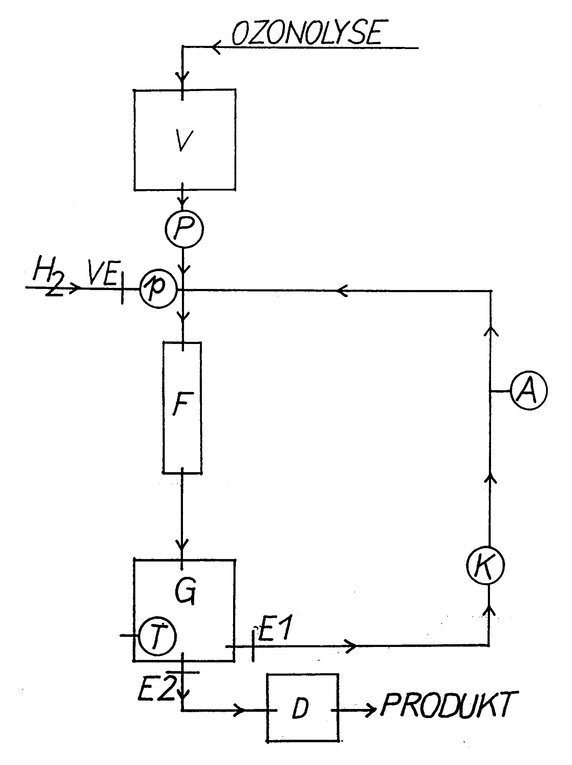

In einer besonders bevorzugten Ausführungsform der Erfindung wird eine Vorrichtung gemäß der Zeichnung eingesetzt. In der Zeichnung bedeutet V einen Vorratsbehälter, der die peroxidischen Ozonolyseprodukte im verwendeten Verdünnungsmittel enthält. Dem Vorratsbehälter V können die peroxidischen Ozonolyseprodukte direkt aus der Ozonolyse zugeführt werden. Die Reaktionslösung wird aus V mittels einer Pumpe P gleichzeitig mit Wasserstoff im Gleichstrom über den Monolithkatalysator F geleitet und gelangt in ein Gefäß G, in dem eine Anordnung T zur Temperaturkontrolle angebracht ist, mit deren Hilfe eine Temperatur von Raumtemperatur bis 60 °C aufrechterhalten wird Der Wasserstoffdruck wird mittels eines Druckmessers p und mittels eines Ventils VE auf 0,1 bis 0,3 MPa eingestellt. Da die Reaktionslösung nach einmaligem Kontakt mit dem Monolithkatalysator F im allgemeinen nicht aushydriert ist, wird sie mittels einer Kreislaufpumpe K aus dem Gefäß G über die Entnahmevorrichtung E1 so lange über den Monolithkatalysator F gepumpt, bis die Peroxidkonzentration in der Reaktionslösung, die über eine Anordnung A gemessen wird, auf eine gewünschte Konzentration abgesunken ist. Der Monolithkatalysator besteht dabei vorteilhafterweise aus einem Metallrohr, in dem sich Waben oder Lamellen befinden, die mit dem Katalysatorgrundstoff beschichtet sind. Das Metallrohr kann dabei so beschaffen sein, daß es direkt in die Reaktionsleitung eingesetzt werden kann, wodurch es möglich ist, den Katalysator schnell und auf einfache Art und Weise auszuwechseln. Durch die Fixierung des Katalysators auf einem Träger kann es dabei zu keiner Sedimentation des Katalysators in den Reaktionsleitungen kommen.

Im Falle, daß destillierbare Hydrogenolyseprodukte hergestellt werden, wird die Reaktionslösung nach erfolgter Reaktion über die Entnahmevorrichtung E2 aus dem Gefäß G einer Destillationsanlage D zugeführt, in der die Hydrogenolyseprodukte aus dem verwendeten Verdünnungsmittel in großer Reinheit und in hohen Ausbeuten isoliert werden. Eine solche Vorrichtung, die zur hydrogenolytischen Reduktion verschiedener chemischer Verbindungen, die durch Hydrogenolyse mit Hilfe eines Monolithkatalysators und Wasserstoff in einheitliche, neue Produkte übergeführt werden können, geeignet ist, ist neu und ebenfalls Gegenstand der Erfindung.

Auf die erfindungsgemäße Art und Weise werden peroxidische Ozonolyseprodukte direkt aus der Ozonolyse auf einfache Art und Weise zu sehr reinen Hydrogenolyseprodukten in hohen Ausbeuten reduziert, wobei keine Base zugesetzt werden muß und wobei gefährliche und komplizierte Operationen zur Abtrennung des Katalysators vermieden werden.
Die Erfindung stellt daher eine Bereicherung der Technik dar.

### Beispiel 1

187 g Maleinsäuremethylester (1,3 Mol) in 1000 ml Methanol wurden durch Einleiten eines Stromes von 1000 l Sauerstoff pro Stunde, der 4 Gew.% Ozon enthielt, bei Temperaturen von -15 bis -10 °C mit der äquivalenten Menge Ozon versetzt, bis der Maleinsäuremethylestergehalt weniger als 1 % der Ausgangskonzentration betrug.

Die dabei entstandene, peroxidhaltige Lösung wurde in Gegenwart eines Monolithkatalysators, der aus einem Stahlrohr, in dem sich Lamellen befanden, die mit insgesamt 7.2 g Platin beschichtet waren, unter einem Wasserstoffdruck von 0,12 MPa hydriert. Die Wasserstoffaufnahme betrug 28,6 Nl, das sind 98,2 % der Theorie. Nachdem die Peroxidkonzentration auf < 10 mMol/l abgesunken war, wurde die Reaktion abgebrochen und der Reaktionslösung nach Abdampfen des Methanols bei 55 °C und 25 Torr destilliert.

Dabei wurden 299,5 g Glyoxylsäuremethylesterhalbacetals, das sind 96 % der Theorie mit einer Reinheit von praktisch 100 % erhalten.

### Beispiel 2

156 g Styrol in etwa 850 ml Methanol wurden in der in Beispiel 1 beschriebenen Art und Weise mit Ozon behandelt, wobei etwa 1 Liter einer 1,5 molaren peroxidischen Ozonolyselösung erhalten wurde, die in einer Vorrichtung gemäß der Zeichnung bei einer Temperatur von 30 bis 40°C und einem Druck von etwa 0,12 MPa in Gegenwart eines metallischen Monolithkatalysators, beschichtet mit einem Lindlar Katalysatorgrundstoff, mit Wasserstoff behandelt wurde. Die Wasserstoffaufnahme betrug 31,9 Nl, das sind 94,9 % der Theorie. Nach erfolgter Reduktion wurde das Verdünnungsmittel abgedampft und der Rückstand vakuumdestilliert. Bei einem Siedepunkt von 119 bis 120°C/14 mm Hg wurden dabei 149,3 g Benzaldehyd, das sind etwa 94 % der Theorie bezogen auf das eingesetzte Styrol mit praktisch 100%iger Reinheit erhalten.

### Beispiele 3 und 4

Wurden auf die im Beispiel 2 beschriebene Art und Weise, aber unter Verwendung von
**3**. 177 g 4-Methylstyrol, wobei 32,4 Nl Wasserstoff, das sind 96,4 % der Theorie, aufgenommen wurden,
**4**. 121,5 g Isosafrol (3,4-Methylendioxy-(alpha-methyl)-styrol), wobei 15,8 Nl Wasserstoff, das sind 97,5 % der Theorie, aufgenommen wurden,
durchgeführt. Dabei wurden bei einem Siedepunkt von
**3**. 106 bis 108°C/10 mm Hg 169 g 4-Methylbenzaldehyd, das sind 94 % der Theorie bezogen auf das eingesetzte 4-Methylstyrol
**4**. 106 bis 107°C/4 mm Hg 140,6 g Heliotropin (3,4-Methylendioxy-benzaldehyd), das sind etwa 94 % der Theorie bezogen auf das eingesetzte Isosafrol
mit praktisch 100%iger Reinheit erhalten.

### Beispiel 5

Wurde auf die im Beispiel 2 beschriebene Art und Weise, aber unter Verwendung von 223,5 g 4-Nitrostyrol, wobei 31,8 Nl Wasserstoff, das sind 94,6 % der Theorie, aufgenommen wurden, durchgeführt. Nach Abdampfen des Verdünnungsmittels wurde der Abdampfrückstand in heissem Wasser gelöst und die Lösung im Eisbad gekühlt. Der ausgefallene Niederschlag wurde abfiltriert und getrocknet. Dabei wurden 216 g 4-Nitrobenzaldehyd, das sind etwa 96 % der Theorie bezogen auf 4-Nitrostyrol mit einem Schmelzpunkt von 105 bis 106 °C erhalten.

### Beispiel 6

Eine 0,5 molare Naphthalinlösung in Methanol wurde wie im Beispiel 1 beschrieben der Ozonolyse unterworfen. Die dabei entstandene Reaktionslösung, die 1 molar an ortho-Phthalaldehydozonolyseprodukten war, wurde gemäß der Zeichnung kontinuierlich über einen Monolithkatalysator unter einem Wasserstoffdruck von 0,12 MPa bei einer Temperatur von 30 bis 35 °C geleitet. Dabei sank die Peroxidkonzentration auf weniger als 10 mMol/l und es wurden 94 % der Theorie an Wasserstoff verbraucht. Aus der Produktlösung, die kontinuierlich abgezogen wurde, wurde das Methanol abgedampf und der Rückstand in so viel heißem Wasser gelöst, daß eine klare Lösung entstand. Beim Stehen in der Kälte kristallisierte ein Teil des entstandenen o-Phthalaldehyds aus. Die wäßrige Phase wurde zwei Mal mit Diethylether extrahiert, der bereits kristallisierte o-Phthalaldehyd in den vereinigten Ätherphasen gelöst und das organische Lösungsmittel abgedampft.

Dabei wurden 88,2 % der Theorie o-Phthalaldehyd mit einer Reinheit von praktisch 100 % und einem Schmelzpunkt von 54 °C erhalten.

### Beispiel 7

Wurde wie Beispiel 6 aber unter Verwendung eines Monolithkatalysators, der mit Palladium beschichtet war, duchgeführt. Die Wasserstoffaufnahme betrug 97 % der Theorie, die Ausbeute an o-Phthalaldehyd 90 % der Theorie in einer Reinheit von praktisch 100 % mit einem Schmelzpunkt von 54 °C.

### Beispiele 8 und 9

Wurden auf die im Beispiel 2 beschriebene Art und Weise, aber unter Verwendung von
**8**. 210 g 1-Decen, wobei 32,4 Nl Wasserstoff, das sind 96,4 % der Theorie, aufgenommen wurden,
**9**. 172 g 1,4-Diacetoxybuten-2, wobei 21,8 Nl Wasserstoff, das sind 97,3 % der Theorie, aufgenommen wurden,
durchgeführt. Dabei wurden bei einem Siedepunkt von
**8**. 79 bis 81°C/12 mm Hg 201 g Nonanal, das sind etwa 94 % der Theorie bezogen auf das eingesetzte 1-Decen
**9**. 55 bis 56°C/15 mm Hg 188 g Acetoxyacetaldehyd, das sind etwa 92 % der Theorie bezogen auf das eingesetzte 1,4-Diacetoxybuten-2
mit praktisch 100%iger Reinheit erhalten.

### Beispiel 10

1 l einer 1,5 molaren Cyclooctadienozonolyseprodukte enthaltenden Methanollösung, hergestellt auf die im Beispiel 1 beschriebene Art und Weise, wurde wie im Beispiel 1 beschrieben, reduziert. Dabei wurden 98 % der Theorie an Wasserstoff aufgenommen. Die Produktlösung wurde zur Charakterisierung acetalisiert und das dabei erhaltene Reaktionsgemisch im Vakuum fraktioniert. Dabei wurden 90,1 % der Theorie 1,1,4,4-Tetramethoxybutan mit einem Siedepunkt von 86 bis 87 °C bei 15 Torr in praktisch 100%iger Reinheit erhalten.

### Beispiel 11

Wurden auf die im Beispiel 6 beschriebene Art und Weise, aber unter Verwendung von
123 g Cyclohexen, wobei die Wasserstoffaufnahme 30,5 Nl, das sind 90,8 % der Theorie, betrug, durchgeführt. Die Produktlösung wurde zur Charakterisierung acetalisiert und das dabei erhaltene Reaktionsgemisch im Vakuum fraktioniert. Dabei wurden 275 g 1,1,6,6-Tetramethoxyhexan, das sind 89 % der Theorie bezogen auf das eingesetzte Cyclohexen mit einem Siedepunkt von 111°C/20 mm Hg in praktisch 100%iger Reinheit erhalten.

### Beispiel 12

Wurde auf die im Beispiel 11 beschriebene Art und Weise, aber unter Verwendung von 105 g 2,5-Dihydrofuran, wobei die Wasserstoffaufnahme 31,9 Nl, das sind 94,9 % der Theorie, betrug, durchgeführt. Eine Oximtitration der Produktlösung ergab einen Aldehydgruppengehalt des gebildeten 3-Oxaglutaraldehyd von 2,88 Mol. Das entspricht einer Ausbeute von 96 % der Theorie bezogen auf das eingesetzte 2,5-Dihydrofuran.

### Beispiele 13 und 14

Wurden auf die im Beispiel 2 beschriebene Art und Weise, aber unter Verwendung von
**13**. 105 g 2-Vinylpyridin, wobei 21,5 Nl Wasserstoff, das sind 96 % der Theorie, aufgenommen wurden,
**14**. 52,5 g 4-Vinylpyridin, wobei 10,2 Nl Wasserstoff, das sind 91 % der Theorie, aufgenommen wurden
durchgeführt. Dabei wurden bei einem Siedepunkt von
**13**. 59 bis 62°C/10 mm Hg 97,4 g Pyridin-2-aldehyd, das sind 91 % der Theorie bezogen auf das eingesetzte 2-Vinylpyridin
**14**. 70 bis 72°C/10 mm Hg 48 g Pyridin-4-aldehyd, das sind etwa 90% der Theorie bezogen auf das eingesetzte 4-Vinylpyridin
mit praktisch 100%iger Reinheit erhalten.

### Beispiele 15 bis 17

Wurden auf die im Beispiel 2 beschriebene Art und Weise, aber unter Verwendung von
**15**. 150 g Methacrylsäuremethacrylat, wobei 32,2 Nl Wasserstoff, das sind 96 % der Theorie, aufgenommen wurden,
**16**. 171 g Methacrylsäureethylacrylat, wobei 31,9 Nl Wasserstoff, das sind 95 % der Theorie, aufgenommen wurden,
**17**. 192 g Äthylacrylsäureethylester, wobei 31,9 Nl Wasserstoff, das sind 95 % der Theorie, aufgenommen wurden,
durchgeführt. Dabei wurden bei einem Siedepunkt von
**15**. 61 bis 62°C/40 mm Hg 145,5 g Brenztraubensäuremethylester, das sind etwa 95 % der Theorie bezogen auf das eingesetzte Methacrylsäuremethacrylat
**16**. 78 bis 80°C/15 mm Hg 164 g Brenztraubensäureethylester, das sind etwa 94 % der Theorie bezogen auf das eingesetzte Methacrylsäureethylacrylat
**17**. 68 bis 69°C/20 mm Hg 178 g 2-Oxo-buttersäureethylester, das sind etwa 92 % der Theorie bezogen auf den eingesetzten Äthylacrylsäureethylester
mit praktisch 100%iger Reinheit erhalten.

## Patentansprüche

1. Verfahren zur hydrogenolytischen Reduktion peroxidischer Ozonolyseprodukte zu den entsprechenden Carbonylverbindungen in einem unter den Reaktionsbedingungen inerten organischen Verdünnungsmittel in Gegenwart eines Katalysators bei einem Wasserstoffdruck von 0,01 bis 2,0 MPa und bei Temperaturen von -10 bis 150 °C, dadurch gekennzeichnet, daß als Katalysator ein Monolithkatalysator eingesetzt wird.

2. Verfahren nach Anspruch 1, dadurch gekennzeichnet, daß der Monolithkatalysator aus einem Katalysatorträger, der mit einem Edelmetall beschichtet ist, besteht.

3. Verfahren nach Anspruch 2, dadurch gekennzeichnet, daß der Katalysatorträger aus einem Metall besteht.

4. Verfahren nach einem der Ansprüche 1 bis 3, dadurch gekennzeichnet, daß als inertes, organisches Verdünnungsmittel ein Alkohol mit 1 bis 6 C-Atomen eingesetzt wird.

5. Verfahren nach einem der Ansprüche 1 bis 4, dadurch gekennzeichnet, daß ein Wasserstoffdruck von 0,1 bis 1,0 MPa aufrechterhalten wird.

6. Verfahren nach einem der Ansprüche 1 bis 5, dadurch gekennzeichnet, daß eine Temperatur von 15 bis 70°C aufrechterhalten wird.

7. Verfahren nach einem der Ansprüche 1 bis 6, dadurch gekennzeichnet, daß eine Vorrichtung enthaltend einen Vorratsbehälter V für die zu hydrogenolysierende chemische Verbindung, eine Pumpe P zur Einspeisung der chemischen Verbindung, eine Einrichtung VE zum Einbringen von Wasserstoff und einen Druckmesser p zum Messen des Wasserstoffdruckes, einen Behälter F mit einem Monolithkatalysator, ein Gefäß G zum Auffangen der Reaktionslösung, das eine Einrichtung T zur Temperaturkontrolle und Einrichtungen E1 und E2 zur Entnahme der Produktlösung enthält, eine Kreislaufpumpe K zur Beförderung der Reaktionslösung und eine Vorrichtung A zur Probenentnahme, wobei der Vorratsbehälter V, die Pumpe P, das Wasserstoffventil VE, der Druckmesser für Wasserstoff p, der Monolithkatalysator F, das Gefäß G, die Kreislaufpumpe K und die Vorrichtung zur Probenentnahme A hintereinander durch Leitungen für die Reaktionslosung verbunden sind, wobei gewährleistet ist, daß die zu hydrogenolysierende chemische Verbindung und der Wasserstoff im Gleichstrom über den Monolithkatalysator F und in das Gefäß G zum Auffangen der Reaktionslösung geleitet werden und sodaß weiterhin gewährleistet ist, daß die Reaktionslösung vom Auffanggefäß mit Hilfe der Kreislaufpumpe erneut über den Monolithkatalysator geleitet werden kann, eingesetzt wird.

## Claims

1. Process for the hydrogenolytic reduction of peroxidic ozonolysis products to give the corresponding carbonyl compounds in an organic diluent which is inert under the reaction conditions in the presence of a catalyst under a hydrogen pressure of 0.01 to 2.0 MPa and at temperatures of -10 to 150°C, characterized in that a monolithic catalyst is employed as the catalyst.

2. Process according to Claim 1, characterized in that the monolithic catalyst comprises a catalyst support coated with a noble metal.

3. Process according to Claim 2, characterized in that the catalyst support comprises a metal.

4. Process according to one of Claims 1 to 3, characterized in that an alcohol having 1 to 6 C atoms is employed as the inert organic diluent.

5. Process according to one of Claims 1 to 4, characterized in that a hydrogen pressure of 0.1 to 1.0 MPa is maintained.

6. Process according to one of Claims 1 to 5, characterized in that a temperature of 15 to 70°C is maintained.

7. Process according to one of Claims 1 to 6, characterized in that a device comprising a reservoir tank V for the chemical compound to be hydrogenolyzed, a pump P for feeding in the chemical compound, a device VE for introducing hydrogen and a manometer P for measuring the hydrogen pressure, a container F with a monolithic catalyst, a vessel G for collecting the reaction solution, which comprises a device T for temperature control and devices E1 and E2 for removal of the product solution, a circulating pump K for conveying the reaction solution and a device A for sampling, the reservoir tank V, the pump P, the hydrogen valve VE, the manometer for hydrogen P, the monolithic catalyst F, the vessel G, the circulating pump K and the device for sampling A being connected in series by lines for the reaction solution, it being ensured that the chemical compound to be hydrogenolyzed and the hydrogen are passed in co-current over the monolithic catalyst F and into the vessel G for collecting the reaction solution, so that it is furthermore ensured that the reaction solution can be passed from the collecting vessel with the aid of the circulating pump over the monolithic catalyst again, is employed.

## Revendications

1. Procédé de réduction hydrogénolytique de produits peroxydiques d'ozonolyse en composés carbonyles correspondants, dans un solvant organique inerte dans les conditions de réaction, en présence d'un catalyseur, à une pression d'hydrogène de 0,01 à 2,0 MPa et à des températures de -10 à 150°C, caractérisé en ce qu'on utilise, comme catalyseur, un catalyseur monolithique.

2. Procédé selon la revendication 1, caractérisé en ce que le catalyseur monolithique est formé d'un support de catalyseur qui est revêtu d'un métal précieux.

3. Procédé selon la revendication 2, caractérisé en ce que le support de catalyseur est formé d'un métal.

4. Procédé selon l'une des revendications 1 à 3, caractérisé en ce qu'on utilise, comme solvant organique inerte, un alcool ayant de 1 à 6 atomes de carbone.

5. Procédé selon l'une des revendications 1 à 4, caractérisé en ce qu'on maintient une pression d'hydrogène de 0,1 à 1,0 MPa.

6. Procédé selon l'une des revendications 1 à 5, caractérisé en ce qu'on maintient une température de 15 à 70°C.

7. Procédé selon l'une des revendications 1 à 6, caractérisé en ce qu'on utilise un dispositif contenant un réservoir V pour le composé chimique à hydrogénolyser, une pompe P pour amener le composé chimique, un dispositif VE pour introduire de l'hydrogène et un manomètre p pour mesurer la pression d'hydrogène, un réservoir F contenant un catalyseur monolithique, une cuve G pour recevoir la solution de réaction, qui contient un dispositif T pour le contrôle de la température et des dispositifs E1 et E2 pour soutirer la solution de produit, une pompe de circulation K pour transporter la solution de réaction et un dispositif A pour prélever des échantillons, le réservoir V, la pompe P, la vanne à hydrogène VE, le manomètre p pour l'hydrogène, le catalyseur monolithique F, la cuve G, la pompe de circulation K et le dispositif A de prélèvement d'échantillons étant reliés à l'arrière par des canalisations pour la solution de réaction, ce dispositif garantissant que le composé chimique à hydrogénolyser et l'hydrogène soient amenés dans le même sens sur le catalyseur monolithique F et dans la cuve G servant à recevoir la solution de reaction, et, de cette manière, garantissant aussi que la solution de réaction puisse être amenée de la cuve de réception de nouveau sur le catalyseur monolithique à l'aide de la pompe de circulation.
